Europäisches Patentamt

European Patent Office

Office européen des brevets

(19)

(11) Veröffentlichungsnummer: **0 083 734**
**B1**

(12) **EUROPÄISCHE PATENTSCHRIFT**

(45) Veröffentlichungstag der Patentschrift:
05.03.86

(21) Anmeldenummer: **82111201.8**

(22) Anmeldetag: **03.12.82**

(51) Int. Cl.⁴: **C 07 K 15/26**, C 07 K   3/24,
A 61 K 45/02

(54) **Kristallines Human-Leukocyten-Interferon.**

(30) Priorität: **07.12.81 US 327876**

(43) Veröffentlichungstag der Anmeldung:
**20.07.83 Patentblatt 83/29**

(45) Bekanntmachung des Hinweises auf die Patenterteilung: **05.03.86 Patentblatt 86/10**

(84) Benannte Vertragsstaaten:
**AT BE CH DE FR GB IT LI LU NL SE**

(56) Entgegenhaltungen:
**Chemical Abstracts Band 96, Nr. 11, 15. März 1982, Columbus, Ohio USA D.L. MILLER et al. "Crystallization of recombinant human leukocyte interferon A", Seite 452, Spalte 2, Abstract Nr. 83929r**
**METHODS IN ENZYMOLOGY, Band 79, Teil 3, 1981, Academic Press, New York D.L. MILLER et al. "The crystallization of recombinant human leukocyte interferon A", Seiten 3-7**

(73) Patentinhaber: **F. HOFFMANN-LA ROCHE & CO. Aktiengesellschaft**

**CH-4002 Basel (CH)**

(72) Erfinder: **Kung, Hsiang-Fu**
**21 West Lincoln Street**
**Verona, N.J. 07044 (US)**
Erfinder: **Miller, David L.**
**99 Essex Avenue**
**Montclair, N.J. 07042 (US)**
Erfinder: **Pestka, Sidney**
**82 Brookside Terrace**
**North Caldwell, N.J. 07006 (US)**

(74) Vertreter: **Lederer, Franz, Dr. et al**
**Patentanwälte Dr. Lederer Franz Meyer-Roxlau Reiner F. Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Humane Leukocyten-Interferone, mit $\alpha_1$, $\alpha_2$, $\beta_1$, $\beta_2$, $\beta_3$, $\gamma_1$, $\gamma_2$, $\gamma_3$, $\gamma_4$ und $\gamma_5$ bezeichnet und aus Virus-induzierten Leukocyten normaler und leukämischer Spender gewonnen, sind bis zur Homogenität gereinigt worden (vgl. U.S.P. 4,289,690 vom 15.9.81). Kürzlich wurde die rekombinante DNS-Technologie zur mikrobiellen Herstellung einer Reihe verschiedener humaner Leukocyten-Interferone (IFN-$\alpha$) mit einer Homologie ihrer Aminosäuresequenzen in der Grössenordnung von 70 % angewandt (vgl. Nature *290*, 20-26 [1981]). Spezifische, in dieser Publikation genannte rekombinante Human-Leukocyten-Interferone (rIFN-$\alpha$) sind rIFN-$\alpha$A, B, C, D, E, F, G und H. In der deutschen Offenlegungsschrift No. 31 25 706 wurden zusätzlich die Spezies rIFN-$\alpha$I und rIFN-$\alpha$J beschrieben. Schliesslich ist in der deutschen Offenlegungsschrfit No. 31 44 469 die mikrobielle Herstellung via rekombinanter DNS-Technologie von hybriden Leukocyten-Interferonen beschrieben, beispielsweise von

rIFN-$\alpha$ $A_{1-91}$ $D_{93-166}$ ; $D_{1-92}$ $A_{92-165}$ ; $A_{1-62}$ $D_{64-166}$ ;

$D_{1-63}$ $A_{63-165}$ ; $A_{1-91}$ $B_{93-166}$ ; $A_{1-91}$ $F_{93-166}$ ;

$A_{1-91}$ $G_{93-166}$ ; $A_{1-150}$ $I_{151-165}$ ; $B_{1-92}$ $A_{92-165}$ ;

$B_{1-92}$ $D_{93-166}$ ; $B_{1-92}$ $F_{93-166}$ ; $B_{1-92}$ $G_{93-166}$ ;

$D_{1-92}$ $B_{93-166}$ ; $D_{1-92}$ $F_{93-166}$ ; $D_{1-92}$ $G_{93-166}$ ;

$F_{1-92}$ $A_{92-165}$ ; $F_{1-92}$ $B_{93-166}$ ; $F_{1-92}$ $D_{93-166}$ ;

$F_{1-92}$ $G_{93-166}$ ; und $I_{1-151}$ $A_{152-166}$.

Die vorstehend genannten natürlichen, rekombinanten und hybriden Human-Leukocyten-Interferone repräsentieren eine Gruppe von Proteinen, die die Eigenschaft besitzen, ihre Zielzellen virusresistent zu machen. Darüberhinaus können diese Interferone die Zellproliferation hemmen und die Immun-Antwort modulieren. Diese Eigenschaften haben dazu geführt, dass rIFN-$\alpha$A und rIFN-$\alpha$D als therapeutische Mittel zur Behandlung viraler Infektionen und neoplastischer Erkrankungen klinisch geprüft werden.

Die Kristallisation einer Substanz ist eines der klassischen Kriterien für Homogenität. Ausserdem kann die Kristallisation selbst einen wertvollen Reinigungsschritt darstellen. Die Verfügbarkeit grösserer IFN-$\alpha$-Kristalle wird schliesslich erlauben, die Tertiärstruktur des Interferons durch Röntgenstrukturanalyse aufzuklären.

Obgleich bisher verschiedene Techniken zur Kristallisation von Proteinen entwickelt worden sind, existiert bis heute noch kein Verfahren, das generell verwendet werden könnte und viele Proteine konnten noch nicht kristallisiert werden. Die Kristallisation von Proteinen ist daher noch immer ein Problem, das sich nicht durch theoretische Ueberlegungen allgemein lösen lässt sondern nur von Fall zu Fall durch Ausprobieren.

Die gängigste Methode besteht dabei im Zusatz eines Kristallisationsmittels zur wässrigen Proteinlösung, üblicherweise eines Salzes, wie Ammoniumsulfat oder -citrat, oder eines organischen Lösungsmittels, wie Ethanol oder 2-Methyl-2,4-pentandiol. Mit derartigen Mitteln konnte IFN-$\alpha$ jedoch nicht kristallisiert werden.

Ein sehr vielseitiges Kristallisationsmittel ist Polyethylenglykol (PEG), das einige Charakteristika der Salze und der organischen Lösungsmittel miteinander verbindet. Vgl. hierzu K. B. Ward et al., J. Mol. Biol. *98*, 161 (1975) und A. McPherson, Jr., J. Biol. Chem. *251.*, 6300 (1976). Es wurde nun gefunden, dass PEG, insbesondere PEG 4000, erfolgreich zur Kristallisation der vorerwähnten Interferone, vorzugsweise von rIFN-$\alpha$A, verwendet werden kann.

Die vorliegende Erfindung betrifft daher Human-Leukocyten-Interferon in kristalliner Form, dieses enthaltende pharmazeutische Präparate und ein Verfahren zu dessen Herstellung, das darin besteht, dass man eine wässrige Interferon-Lösung mit einer Konzentration von mindestens etwa 0,2 mg/ml mit PEG behandelt.

Die als Ausgangsmaterial für das erfindungsgemässe Kristallisationsverfahren dienenden Interferone können nach bekannten Verfahren erhalten werden, bei denen sie in im wesentlichen homogener Form anfallen. Zu diesen Verfahren gehören die HPLC (s. U.S.P. 4,289,690), die Affinitätschromatographie an monoklonalen IFN-$\alpha$-Antikörpern auf einem Trägermaterial (s. z. B. T. Staehelin et al., J. Biol. Chem. *256.*, 9750-9754 [1981]) und andere Methoden, die das Human-Interferon in genügender Reinheit ($> 95$ %) und Konzentration ($\geqslant 0,2$ mg/ml) liefern.

Es gibt mehrere Gründe für die Bedeutung, die die vorliegende Erfindung besitzt. Wie bereits oben erwähnt wurde, stellt die Kristallisation einen zusätzlichen Reinigungsschritt dar, bei dem Verunreinigungen, die mittels HPLC oder konventioneller Säulenchromatographie nicht entfernt werden können, eliminiert werden. Darüberhinaus kann kristallines Interferon bei Zimmertemperatur aufbewahrt und versandt werden, da es keine Protease-Verunreinigungen mehr enthält. Schliesslich kann auf diese Weise eine teilweise Denaturierung, die mit einem Aktivitätsverlust verbunden ist und die beispielsweise bei Lyophilisation beobachtet wird, vermieden werden.

Im folgenden Beispiel ist die Kristallisation eines Human-Leukocyten-Interferons, nämlich des rIFN-$\alpha$A, beschrieben. In analoger Weise können andere Human-Leukocyten-Interferone mit einem hohen Grad von Sequenzhomologie kristallisiert werden.

### Beispiel

rIFN-$\alpha$A (4 ml, 0,2 mg/ml) wurde bei 4 °C über Nacht gegen 0,01M wässrige HEPES (N-2-Hydroxyethylpiperazin-N'-2-ethan-sulfonsäure), mit $NH_4OH$ auf pH 7,1 eingestellt, dialysiert. Die Lösung wurde fünfmal durch Eindampfen konzentriert und gegen 0,01M HEPES (pH 7,1) redialysiert bis zu einer Konzentration von 5 mg/ml. Die Endkonzentration wurde UV-spektrophotometrisch ermittelt.

Eine Glastüpfelplatte mit 9 Vertiefungen wurde durch Eintauchen in eine 5 %ige (V/V) Lösung von Dimethyldichlorsilan in $CCl_4$ und anschliessendes Erhitzen auf 180 °C siliconiert. Sie wurde mit Wasser gewaschen und nochmals erhitzt. In jede von 4 Vertiefungen wurden 20 μl Interferonlösung gegeben und soviel einer $NaN_3$-haltigen (0,5 mg/ml) PEG 4 000-Lösung (200 mg/ml), dass die Endkonzentrationen an PEG jeweils 20, 40, 60 und 100 mg/ml betrugen. Die Tröpfchen wurden sofort mit einer Mikropipette verrührt und die Tüpfelplatte wurde über eine Lösung von PEG 4 000 (100 mg/ml) in eine Kristallisationsschale gebracht. Die Schale wurde versiegelt und bei 4 °C aufbewahrt. Nach 1-3 Tagen hatten sich in allen 4 Vertiefungen Kristalle gebildet, die sich mit der Zeit teilweise noch vergrösserten.

Die Kristalle wurden von den flüssigen Phasen durch Zentrifugieren abgetrennt, mit 0,05M HEPES (pH 7,1), enthaltend 10 % PEG 4 000, gewaschen und in 0,05M HEPES (pH 7,1) gelöst. Die Bioassays dieser Lösungen zeigten, dass die Kristalle Interferon-Aktivität aufwiesen und zwar über 90 % der Aktivität der ursprünglichen Kristalle. Gel-elektrophoretisch ergab sich kein Unterschied zwischen kristallinem und nicht-kristallinem rIFN-αA.

Die folgende Tabelle fasst die Ergebnisse der Kristallisation von rIFN-αA bei verschiedenen pH-Werten zusammen :

Tabelle

| | Kristalle | | Ueberstand | |
|---|---|---|---|---|
| | Antivirale Aktivität | | Antivirale Aktivität | |
| | (E/ml) | (%) | (E/ml) | (%) |
| pH 5 | $4,0 \times 10^8$ | 94 | $2,5 \times 10^7$ | 6 |
| pH 7 | $6,0 \times 10^8$ | 99 | $4,5 \times 10^6$ | 1 |
| pH 8 | $8,0 \times 10^8$ | 99 | $4,5 \times 10^6$ | 1 |

Es ist nicht erforderlich, das Interferon aus konzentrierten Lösungen zu kristallisieren. Mikrokristalle treten bereits in Lösungen von 0,2 mg/ml Interferon, 10 % PEG auf. Diese Methode kann daher auch zur Konzentrierung von verdünnten Interferon-Lösungen verwendet werden. Wie aus der obigen Tabelle hervorgeht, liefert die Kristallisation unter schwach basischen Bedingungen, d. h. bei pH 8, kristallines Interferon in quantitativer Ausbeute und mit der höchsten spezifischen Aktivität.

Die Human-Leukocyten-Interferone in kristalliner Form können in der gleichen Weise in pharmazeutischen Präparaten verwendet werden wie die entsprechenden bekannten, gereinigten und homogenen natürlichen, rekombinanten und hybriden Interferone.

**Patentansprüche** (für die Vertragsstaaten : BE, CH, DE, FR, GB, IT, LI, LU, NL, SE)

1. Human-Leukocyten-Interferon (IFN-α) in kristalliner Form.

2. Interferon gemäss Anspruch 1, dadurch gekennzeichnet, dass es sich um natürliches IFN-α handelt.

3. Interferon gemäss Anspruch 1, dadurch gekennzeichnet, dass es sich um rekombinantes IFN-α handelt.

4. Interferon gemäss Anspruch 1, dadurch gekennzeichnet, dass es sich um ein hybrides rekombinantes IFN-α handelt.

5. Rekombinantes Human-Leukocyten-Interferon A (rIFN-αA) in kristalliner Form.

6. Interferon gemäss einem der Ansprüche 1-5 als pharmazeutischer Wirkstoff.

7. Verfahren zur Herstellung von Human-Leukocyten-Interferon in kristalliner Form, dadurch gekennzeichnet, dass man eine wässrige Lösung des Interferons mit einer Konzentration von mindestens etwa 0,2 mg/ml mit Polyethylenglykol (PEG) behandelt.

8. Verfahren gemäss Anspruch 7, dadurch gekennzeichnet, dass es sich um natürliches IFN-α handelt.

9. Verfahren gemäss Anspruch 7, dadurch gekennzeichnet, dass es sich um ein rekombinantes IFN-α handelt.

10. Verfahren gemäss Anspruch 7, dadurch gekennzeichnet, dass es sich um ein hybrides rekombinantes IFN-α handelt.

0 083 734

11. Verfahren gemäss Anspruch 7, dadurch gekennzeichnet, dass es sich um rekombinantes Human-Leukocyten-Interferon (rIFN-αA) handelt.

12. Verfahren gemäss Anspruch 11, dadurch gekennzeichnet, dass man PEG 4 000 verwendet und dessen Endkonzentration 20-100 mg/ml beträgt.

13. Verfahren gemäss Anspruch 12, dadurch gekennzeichnet, dass der pH-Wert der Lösung während der Kristallisation etwa 8,0 beträgt.

14. Verfahren gemäss Anspruch 12, dadurch gekennzeichnet, dass die Konzentration des Interferons in der Ausgangslösung etwa 5,0 mg/ml beträgt.

15. Pharmazeutische Präparate enthaltend Human-Leukocyten-Interferon in kristalliner Form als aktiven Bestandteil.


**Patentansprüche** (für den Vertragsstaat AT)

1. Verfahren zur Herstellung von Human-Leukocyten-Interferon in kristalliner Form, dadurch gekennzeichnet, dass man eine wässrige Lösung des Interferons mit einer Konzentration von mindestens etwa 0,2 mg/ml mit Polyethylenglykol (PEG) behandelt.

2. Verfahren gemäss Anspruch 1, dadurch gekennzeichnet, dass es sich um natürliches IFN-α handelt.

3. Verfahren gemäss Anspruch 1, dadurch gekennzeichnet, dass es sich um ein rekombinantes IFN-α handelt.

4. Verfahren gemäss Anspruch 1, dadurch gekennzeichnet, dass es sich um ein hybrides rekombinantes IFN-α handelt.

5. Verfahren gemäss Anspruch 1, dadurch gekennzeichnet, dass es sich um rekombinantes Human-Leukocyten-Interferon (rIFN-αA) handelt.

6. Verfahren gemäss Anspruch 5, dadurch gekennzeichnet, dass man PEG 4 000 verwendet und dessen Endkonzentration 20-100 mg/ml beträgt.

7. Verfahren gemäss Anspruch 6, dadurch gekennzeichnet, dass der pH-Wert der Lösung während der Kristallisation etwa 8,0 beträgt.

8. Verfahren gemäss Anspruch 6, dadurch gekennzeichnet, dass die Konzentration des Interferons in der Ausgangslösung etwa 5,0 mg/ml beträgt.


**Claims** (for the Contracting States : BE, CH, DE, FR, GB, IT, LI, LU, NL, SE)

1. Human leukocyte interferon (IFN-α) in crystalline form.

2. Interferon in accordance with claim 1, characterized in that it is natural IFN-α.

3. Interferon in accordance with claim 1, characterized in that it is recombinant IFN-α.

4. Interferon in accordance with claim 1, characterized in that it is a hybrid recombinant IFN-α.

5. Recombinant human leukocyte interferon A (rIFN-αA) in crystalline form.

6. Interferon in accordance with any one of claims 1-5 as a pharmaceutically active substance.

7. A process for the manufacture of human leukocyte interferon in crystalline form, characterized by treating an aqueous solution of the interferon at a concentration of at least about 0.2 mg/ml with polyethylene glycol (PEG).

8. A process in accordance with claim 7, characterized in that the interferon is natural IFN-α.

9. A process in accordance with claim 7, characterized in that the interferon is a recombinant IFN-α.

10. A process in accordance with claim 7, characterized in that the interferon is a hybrid recombinant IFN-α.

11. A process in accordance with claim 7, characterized in that the interferon is recombinant human leukocyte interferon (rIFN-αA).

12. A process in accordance with claim 11, characterized in that PEG 4 000 is used and its final concentration is 20-100 mg/ml.

13. A process in accordance with claim 12, characterized in that the pH value of the solution during the crystallization is about 8.0.

14. A process in accordance with claim 12, characterized in that the concentration of the interferon in the starting solution in about 5.0 mg/ml.

15. A pharmaceutical preparation containing human leukocyte interferon in crystalline form as the active ingredient.


**Claims** (for the Contracting State AT)

1. A process for the manufacture of human leukocyte interferon in crystalline form, characterized by treating an aqueous solution of the interferon at a concentration of at least about 0.2 mg/ml with polyethylene glycol (PEG).

4

2. A process in accordance with claim 1, characterized in that the interferon is natural IFN-α.

3. A process in accordance with claim 1, characterized in that the interferon is a recombinant IFN-α.

4. A process in accordance with claim 1, characterized in that the interferon is a hybrid recombinant IFN-α.

5. A process in accordance with claim 1, characterized in that the interferon is recombinant human leukocyte interferon (rIFN-αA).

6. A process in accordance with claim 5, characterized in that PEG 4 000 is used and its final concentration is 20-100 mg/ml.

7. A process in accordance with claim 6, characterized in that the pH value of the solution during the crystallization is about 8.0.

8. A process in accordance with claim 6, characterized in that the concentration of the interferon in the starting solution is about 5.0 mg/ml.

**Revendications** (pour les Etats contractants : BE, CH, DE, FR, GB, IT, LI, LU, NL, SE)

1. Interféron de leucocytes humains (IFN-α) sous forme cristalline.

2. Interféron selon la revendication 1, caractérisé en ce qu'il s'agit d'IFN-α naturel.

3. Interféron selon la revendication 1, caractérisé en ce qu'il s'agit d'IFN-α recombinant.

4. Interféron selon la revendication 1, caractérisé en ce qu'il s'agit d'un IFN-α recombinant hybride.

5. Interféron de leucocytes humains recombinants A (rIFN-αA) sous forme cristalline.

6. Interféron selon l'une des revendications 1-5 comme substance active pharmaceutique.

7. Procédé de préparation d'interféron de leucocytes humains sous forme cristalline, caractérisé en ce qu'on traite une solution aqueuse de l'interféron avec une concentration d'au moins environ 0,2 mg/ml avec du polyéthylèneglycol (PEG).

8. Procédé selon la revendication 7, caractérisé en ce qu'il s'agit d'IFN-α naturel.

9. Procédé selon la revendication 7, caractérisé en ce qu'il s'agit d'un IFN-α recombinant.

10. Procédé selon la revendication 7, caractérisé en ce qu'il s'agit d'un IFN-α recombinant hybride.

11. Procédé selon la revendication 7, caractérisé en ce qu'il s'agit d'un interféron de leucocytes humains (rIFN-α) recombinant.

12. Procédé selon la revendication 11, caractérisé en ce qu'on utilise du PEG 4 000 et en ce que sa concentration finale s'élève à 20-100 mg/ml.

13. Procédé selon la revendication 12, caractérisé en ce que le pH de la solution pendant la cristallisation s'élève à environ 8,0.

14. Procédé selon la revendication 12, caractérisé en ce que la concentration de l'interféron dans la solution de départ s'élève à environ 5,0 mg/ml.

15. Préparations pharmaceutiques contenant de l'interféron de leucocytes humains sous forme cristalline comme composant actif.


**Revendications** (pour l'Etat contractant AT)

1. Procédé de préparation d'interféron de leucocytes humains sous forme cristalline, caractérisé en ce qu'on traite une solution aqueuse de l'interféron à une concentration d'au moins environ 0,2 mg/ml avec du polyéthylèneglycol (PEG).

2. Procédé selon la revendication 1, caractérisé en ce qu'il s'agit d'IFN-α naturel.

3. Procédé selon la revendication 1, caractérisé en ce qu'il s'agit d'un IFN-α recombinant.

4. Procédé selon la revendication 1, caractérisé en ce qu'il s'agit d'un IFN-α recombinant hybride.

5. Procédé selon la revendication 1, caractérisé en ce qu'il s'agit d'un interféron de leucocytes humains recombinants (rIFN-αA).

6. Procédé selon la revendication 5, caractérisé en ce qu'on utilise le PEG 4 000 et en ce que sa concentration finale s'élève à 20-100 mg/ml.

7. Procédé selon la revendication 6, caractérisé en ce que le pH de la solution pendant la cristallisation s'élève à environ 8,0.

8. Procédé selon la revendication 6, caractérisé en ce que la concentration de l'interféron dans la solution de départ s'élève à environ 5,0 mg/ml.